# EUROPÄISCHE PATENTSCHRIFT

(11) **EP 0 718 275 B1**
(45) Veröffentlichungstag und Bekanntmachung des Hinweises auf die Patenterteilung: **26.11.1997**
(21) Anmeldenummer: 95116473.0
(22) Anmeldetag: 19.10.1995
(51) Int. Cl.: C07C 213/06, C07C 219/06, C07C 219/08, C11D 1/62

(54) **Quaternierte Triethanolaminfettsäureester**
Quaternary fatty-acid triethanolamine esters
Esters de triéthanolamine d'acide gras quaternaires

(30) Priorität: 23.12.1994 DE 4446137
(43) Veröffentlichungstag der Anmeldung: 26.06.1996
(73) Patentinhaber: HÜLS AKTIENGESELLSCHAFT, 45764 Marl (DE)
(72) Erfinder: Brock, Michael, Dr., D-46514 Schermbeck (DE); Enneking, Meinolf, D-44627 Herne (DE); Kosswig, Kurt, Dr., D-45772 Marl (DE)

(56) Entgegenhaltungen:
- EP-A- 0 295 385
- EP-A- 0 498 050
- EP-A- 0 550 361
- EP-A- 0 604 726
- WO-A-91/01295
- DE-A- 4 243 701
- US-A- 3 915 867

## Beschreibung

Die Erfindung betrifft quaternierte Triethanolamindifettsäureester hergestellt aus Triethanolamin, Fettsäure, Fettsäureester und einem Quaternierungsmittel, ein Verfahren zu ihrer Herstellung und ihre Verwendung als Komponente in Wäscheweichspülmitteln.

Quaternierte Difettsäureester des Triethanolamins, insbesondere quaternierte Triethanolaminditalgfettsäureester (im folgenden mit TEA-Quat abgekürzt) ersetzen zunehmend das lange Zeit verwendete Ditalgdimethylammoniumchlorid (DTDMAC) als Aktivkomponente in Wäscheweichspülmitteln, da sie sich durch ein günstigeres ökotoxikologisches Verhalten und eine hervorragende biologische Abbaubarkeit auszeichnen (R. Puchta et al., 3. CESIO-Welt-Tensid-Kongreß, London 1992, Band "Proceedings Section D", S. 122). Mit TEA-Quats behandelte Textilien, vor allem Naturfasern wie Baumwolle, sind besser wiederbenetzbar als solche, die mit DTDMAC behandelt worden sind (WO 91/01295). Weiterhin ermöglicht der Einsatz der TEA-Quats die Herstellung von Wäscheweichspülmitteln, deren Aktivgehalte höher als die von DTDMAC enthaltenden Wäscheweichspülmitteln sind.

Quaternierte Triethanolaminfettsäureester können durch Umsetzung von Triethanolamin mit Fettsäuren (WO 91/01295 sowie darin zitierte Patentanmeldungen) oder Fettsäureestern und anschließende Quaternierung der erhaltenen Triethanolaminfettsäureester erhalten werden. Als Fettsäuren werden üblicherweise Talgfettsäure (ungehärtet oder teilgehärtet), als Fettsäureester Talg, also Talgfettsäuretriglycerid (EP 0 284 036) oder säuremethylester (US 3 915 867) verwendet. Quaternierungsmittel ist üblicherweise Dimethylsulfat. Eine besondere Bedeutung kommt dabei Produkten zu, die durch Umsetzung von Triethanolamin mit ca. 2 Äquivalenten eines Talgfettacylrestes und nachfolgender Quaternierung erhalten werden. Es handelt sich hierbei um ein Produktgemisch aus quaternierten Mono-, Di- und Triestern des Triethanolamins und nicht umgesetzten Triethanolamin. Dieses Gemisch ist im weiteren Sinn unter quaterniertem Triethanolamindi(talg)fettsäureester zu verstehen.

Es ist bekannt, daß quaternierte Triethanolamindifettsäureester auf Basis von Ölsäure ebenso dünnflüssig sind wie daraus hergestellte konzentrierte Weichspülmittel, aber über keine textilweichmachende Eigenschaft verfügen (US 3 915 867). Andererseits ist die Textilweichmachung analoger Verbindungen auf Basis einer gesättigten, also gehärteten Talgfettsäure sehr ausgeprägt (DE 16 19 058 oder DE 17 94 068), doch lassen sich daraus nur niedrigkonzentrierte Wäscheweichspülmittel herstellen. Als Kompromiß werden daher marktgängige TEA-Quats aus teilgehärteter Talgfettsäure hergestellt, aus denen sich aber nur lager- und viskositätsstabile Wäscheweichspülmittel mit Aktivgehalten bis zu 20 Gew.-% herstellen lassen.

Die Vermarktung und Verwendung von konzentrierten Wäscheweichspülmitteln bietet aber gegenüber verdünnten Formulierungen für Hersteller und Verbraucher folgende Vorteile:
- Geringeres zu transportierendes Gewicht und Volumen,
- Verminderung der Abfüllkosten,
- Vergrößerung der Herstellkapazität und
- Verringerung des Verpackungsabfalls.
So werden heute verstärkt Anstrengungen unternommen, konzentrierte Wäscheweichspülmittel zu entwickeln.

Ein Wäscheweichspülmittel muß folgende Anforderungen erfüllen, um im Markt erfolgreich plaziert werden zu können:
- Hohe Lagerstabilität,
- hohe Temperaturstabilität,
- hohe Viskositätsstabilität und
- hohe Farbstabilität.

Die Lagerstabilität des Wäscheweichspülmittels muß über einen Zeitraum von mehreren Wochen garantiert sein, d.h. daß es zu keinen Separationserscheinungen kommen darf. Die Konsistenz des Wäscheweichspülmittels darf auch nach großen Temperaturschwankungen nicht verändert sein. Dies gilt sowohl für die Viskosität innerhalb eines angemessenen Rahmens als auch für die Farbe.

Diese Anforderungen werden auch von Wäscheweichspülmitteln, die TEA-Quat enthalten, nur im eingeschränkten Maße erfüllt. Dies gilt vor allem für Formulierungen mit einem Gehalt an TEA-Quat von mehr als 20 Gew.-%. Sie werden innerhalb weniger Tage bei starken Temperaturschwankungen dickflüssig und sind unter Umständen nicht mehr fließfähig.

Aufgabe der vorliegenden Erfindung war es, quaternierte Triethanolamindifettsäureester bereitzustellen, mit denen auch Wäscheweichspülmittel mit Aktivgehalten von über 20 Gew.-% hergestellt werden können, die lager-, temperatur- und viskositätsstabil sind und die textilweichmachend wirken.

Überraschenderweise wurde gefunden, daß mit quaternierten Triethanolamindifettsäureestern auf Basis von Fettsäure/Fettsäureester-Mischungen die gestellte Aufgabe gelöst wird.

Die Erfindung betrifft daher quaternierte Triethanolamindifettsäureester erhalten durch Umsetzung von Triethanolamin mit 1,5 bis 2,5 Äquivalenten einer Mischung aus 70 bis 90 Gew.-% Fettsäure und 10 bis 30 Gew.-% Fettsäureester, jeweils bezogen auf die Gesamtmenge Fettsäure und Fettsäureester, und anschließender Quaternierung des Reaktionsproduktes mit einem Quaternierungsmittel, oder durch getrennte Umsetzung von Triethanolamin mit 1,5 bis 2,5 Äquivalenten Fettsäure und Fettsäureester und Mischung der jeweils erhaltenen Triethanolamindifettsäureester vor der Quaternierung im Gewichtsverhältnis 70 bis 90 % zu 10 bis 30 % und anschließender Quaternierung, oder durch getrennte Umsetzung von Triethanolamin mit 1,5 bis 2,5 Äquivalenten Fettsäure und Fettsäureester und Quaternierung mit einem Quaternierungsmittel und Mischung im Gewichtsverhältnis 70 bis 90 % zu 10 bis 30 %.

Weiterhin betrifft die Erfindung ein Verfahren zur Herstellung von quaternierten Triethanolamindifettsäureestern aus Triethanolamin, Fettsäure, Fettsäureester und einem Quaternierungsmittel, dadurch gekennzeichnet, daß entweder Triethanolamin mit 1,5 bis 2,5 Äquivalenten einer Mischung aus 70 bis 90 Gew.-% Fettsäure und 10 bis 30 Gew.-% Fettsäureester, jeweils bezogen auf die Gesamtmenge Fettsäure und Fettsäureester, umgesetzt und das Reaktionsprodukt anschließend quaterniert wird, oder daß Triethanolamin mit 1,5 bis 2,5 Äquivalenten Fettsäure und Fettsäureester getrennt umgesetzt wird und dann die jeweils erhaltenen Triethanolamindifettsäureester vor der Quaternierung im Gewichtsverhältnis 70 bis 90 % zu 10 bis 30 % gemischt und anschließend quaterniert werden, oder daß Triethanolamin mit 1,5 bis 2,5 Äquivalenten Fettsäure und Fettsäureester getrennt umgesetzt wird und nach der jeweiligen Quaternierung im Gewichtsverhältnis 70 bis 90 % zu 10 bis 30 % gemischt wird.

Weiterhin betrifft die Erfindung die Verwendung der so hergestellten quaternierten Triethanolamindifettsäureester als Komponente in Wäscheweichspülmitteln.

Als besonders geeignet haben sich TEA-Quats erwiesen, die wie folgt hergestellt wurden:
1. Umsetzung von Triethanolamin mit 1,5 bis 2,5, bevorzugt 1,5 bis 2,2, insbesondere 1,5 bis 1,9 Äquivalenten einer Mischung, bestehend aus 70 bis 90 Gew.-% Fettsäure und 10 bis 30 Gew.-% Fettsäureester, jeweils bezogen auf die Gesamtmenge Fettsäure und Fettsäureester, und anschließende Quaternierung.
2. Getrennte Umsetzung von Triethanolamin mit jeweils 1,5 bis 2,5, bevorzugt 1,5 bis 2,2, insbesondere 1,5 bis 1,9 Äquivalenten Fettsäure (Ansatz 1) bzw. Fettsäureester (Ansatz 2) zum Triethanolamindifettsäureester. Beide Ansätze werden dergestalt vereinigt, daß die Mischung aus 70 bis 90 Gew.-% des Ansatzes 1 und 10 bis 30 Gew.-% des Ansatzes 2 besteht. Anschließend wird diese Mischung quaterniert.
3. Getrennte Umsetzung von Triethanolamin mit jeweils 1,5 bis 2,5, bevorzugt 1,5 bis 2,2, insbesondere 1,5 bis 1,9 Äquivalenten Fettsäure (Ansatz 3) bzw. Fettsäureester (Ansatz 4) zum Triethanolamindifettsäureester und getrennte Quaternierung. Beide Ansätze werden dergestalt vereinigt, daß die Mischung aus 70 bis 90 Gew.-% des TEA-Quats des Ansatzes 3 und 10 bis 30 Gew.-% des TEA-Quats aus Ansatz 4 besteht.

Zur Herstellung der Triethanolamindifettsäureester und der TEA-Quats nach 1. bis 3. werden die üblichen und an sich bekannten Reaktionsbedingungen gewählt (WO 91/01295, US 3 915 867, EP 0 284 036 und EP-B 0 295 385).

Die erfindungsgemäß einzusetzenden Fettsäuren können natürlichen oder synthetischen Ursprungs sein. Zweckmäßigerweise kommen solche in Frage, die 6 bis 24, vorzugsweise 8 bis 22, insbesondere 10 bis 20 Kohlenstoffatome enthalten. Die Fettsäuren natürlichen Ursprungs leiten sich z. B. von Olivenöl, Sonnenblumenöl, Palmöl, Rapsöl, Rizinusöl, Sesamöl, Talg, Fischöl usw. ab, wobei auch deren hydrierte oder teilhydrierte Derivate Verwendung finden. Weitere Fettsäuren, aus denen die erfindungsgemäßen quaternierten Triethanolamindifettsäureester hergestellt werden können, sind Caprinsäure, Caprylsäure, Capronsäure, Laurinsäure, Mryistinsäure, Palmitinsäure, Stearinsäure, Ölsäure, Elaidinsäure, Linolsäure, Linolensäure, Behensäure, Erucasäure und Rizinusölsäure. Bei den Fettsäuren synthetischen Ursprungs sind alle vorgenannten geradkettigen Komponenten sowie die verzweigtkettigen Typen erwähnenswert, wie beispielsweise solche, die aus der Oxo-Synthese stammen, und weiterhin Isostearinsäure oder aber Derivate aus der Guerbet-Kondensation.
Besonders bevorzugt ist ungehärtete und teilgehärtete Talgfettsäure.

Die erfindungsgemäß einzusetzenden Fettsäureester entstammen den genannten Fettsäuren, wobei die Alkohole der Fettsäureester 1- bis 6-wertige Alkohole mit 1 bis 8, vorzugsweise 1 bis 6, insbesondere 1 bis 3 Kohlenstoffatomen, sind.

Besonders bevorzugt ist ungehärteter und teilgehärteter Talgfettsäuremethylester.

Die Quaternierung erfolgt üblicherweise mit Dimethylsulfat.

Die quaternierten Triethanolamindifettsäureester werden erfindungsgemäß in Mischungen mit Lösungsmitteln als Rohstoff zu Wäscheweichspülmitteln weiterverarbeitet. Es können Lösungsmittel gemäß Formel I

C₁-C₄-Alkyl-OH (I)

oder Formel II 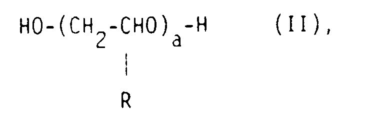 wobei R = H, CH₃ und a = 1 bis 4, oder alkoxylierte natürliche Öle und Fette gemäß DE-A 42 15 689 und EP-A 0 604 726, verwendet werden.

### Beispiele

Die folgenden Beispiele sollen die erfindungsgemäße Herstellung und Verwendung veranschaulichen; sie haben jedoch in keinem Fall einschränkenden Charakter.
Für die Untersuchungen der nachfolgend aufgeführten Beispiele werden quaternierte Triethanolaminditalgfettsäureester (Verbindungen 1 bis 3) gewählt, die sich in der eingesetzten Rohstoffbasis unterscheiden:
◆ Verbindung 1: Produkt aus der Umsetzung von teilgehärteter Talgfettsäure (C-Kettenverteilung der Acylreste: s. Tabelle 1) mit Triethanolamin (Veresterungsgrad: 1,5) und anschließender Quaternierung mit Dimethylsulfat. Verbindung 1 liegt als Mischung in 10 Gew.-% Isopropanol vor.
◆ Verbindung 2: Produkt aus der Umsetzung von nicht gehärtetem Talgfettsäuremethylester (C-Kettenverteilung der Acylreste: s. Tabelle 1) mit Triethanolamin (Veresterungsgrad: 1,5) und anschließender Quaternierung mit Dimethylsulfat. Verbindung 2 liegt als Mischung in 10 Gew.-% Isopropanol vor.
◆ Verbindung 3: Produkt aus der Umsetzung von teilgehärtetem Talgfettsäuremethylester (C-Kettenverteilung der Acylreste: s. Tabelle 1) mit Triethanolamin (Veresterungsgrad: 1,5) und anschließender Quaternierung mit Dimethylsulfat. Verbindung 3 liegt als Mischung in 10 Gew.-% Isopropanol vor.

**Tabelle 1:**

| C-Kettenverteilungen der Acylreste in den Verbindungen 1 bis 3 (Angaben in Gew.-%, bezogen auf den Gesamtgehalt der Acylreste) | | | |
|---|---|---|---|
| **Acylrest** | **Verbindung** | | |
| | **1** | **2** | **3** |
| C₁₄ | 3 | 1 | 1 |
| C₁₆ | 28 | 25 | 28 |
| C₁₆-en | 2 | 3 | 2 |
| C₁₈ | 29 | 8 | 31 |
| C₁₈-en | 37 | 55 | 37 |
| C₁₈-dien | 1 | 9 | 1 |
| Anteil ungesättigter Acylrest | 40 | 67 | 40 |

Die Verbindungen und 2 werden in folgenden Gewichtsverhältnissen miteinander gemischt:
◆ Mischung 1: 100 % Verbindung 1;
◆ Mischung 2: 75 % Verbindung 1 und 25 % Verbindung 2;
◆ Mischung 3: 67 % Verbindung 1 und 33 % Verbindung 2;
◆ Mischung 4: 50 % Verbindung 1 und 50 % Verbindung 2;
◆ Mischung 5: 100 % Verbindung 2.

Orientierend wird aus den 5 Mischungen jeweils ein Weichspülmittel mit 20 Gew.-% Aktivgehalt hergestellt und rheologisch untersucht. Folgende Rezeptur wird verwendet (Angaben in Gew.-%):

| | |
|---|---|
| Mischung 1 bis 5 | 22,2 |
| 25%ige wäßrige CaCl₂-Lösung | 1,2 |
| Parfüm | 0,4 |
| Rest zu 100 Gew.-% Wasser | |

Anschließend werden die Viskositäten der Weichspülmittel direkt nach der Herstellung und nach 2 Wochen Lagerung bei 25 °C nach Brookfield bei 25 °C bestimmt.

| | **Weichspülmittel basierend auf Mischung** | | | | |
|---|---|---|---|---|---|
| | **1** | **2** | **3** | **4** | **5** |
| Viskosität sofort [mPa·s] | 650 | 100 | 130 | 40 | 1 100 |
| Viskosität nach 2 Wochen bei 25 °C [mPa·s] | - | 160 | - | - | - |

Die mit "-" gekennzeichneten Stellen deuten an, daß bei den angegebenen Testbedingungen die Weichspülmittel entweder inhomogen werden oder so hochviskos sind, daß die Viskosität nicht bestimmt werden kann.

Das Weichspülmittel auf Basis der Mischung 5 ist schon direkt nach der Herstellung sehr hochviskos. Nach der Lagerung dickt dieses Weichspülmittel so stark ein, daß die Viskosität nicht mehr bestimmt werden kann. Dies überrascht, da das Weichspülmittel mit Mischung 5 (= Verbindung 2) den von allen Mischungen höchsten Anteil an ungesättigten Acylresten enthält. Damit steht das Ergebnis der allgemeinen Ansicht entgegen, daß ein hoher Anteil von ungesättigten Acylresten im TEA-Quat im Gegensatz zu einem TEA-Quat mit einem hohen Anteil an gesättigten Acylresten automatisch zu niedrigviskoseren Weichspülmitteln führen muß. Dies kann nur mit dem wichtigen Umstand erklärt werden, daß die Verbindung 2 ausgehend von einem Methylester hergestellt worden ist.

Das Weichspülmittel auf Basis Mischung 3 bzw. 4 ist zwar direkt nach der Herstellung niedrigviskos, aber nicht lagerstabil. Es treten Inhomogenitäten auf.

Anfangs hochviskos und während der Lagerung stark eindickend verhält sich das auf Mischung 1 (= Verbindung 1) basierende Weichspülmittel.

Vorteilhaft verhält sich das Weichspülmittel auf Basis der erfindungsgemäßen Mischung 2 (75 Gew.-% Verbindung 1 und 25 Gew.-% Verbindung 2), das auch nach 2 Wochen Lagerung bei 25 °C sehr niedrigviskos bleibt. Um ausschließen zu können, daß nur der Anteil der ungesättigten Acylreste in Mischung 2 für die gute Lagerstabilität verantwortlich ist, wird eine der Mischung 2 analoge Mischung 6 hergestellt, die statt der Verbindung 2 Verbindung 3 enthält. Mischung 6 hat somit die folgende Zusammensetzung:
◆ Mischung 6: 75 Gew.-% Verbindung 1 und 25 Gew.-% Verbindung 3
Diese erfindungsgemäße Mischung 6 wird neben der erfindungsgemäßen Mischung 2 und Mischung 1 in einer weiteren Untersuchungsreihe dahingehend geprüft, ob daraus auch unter strengeren Versuchsbedingungen niedrigviskose und lagerstabile Weichspülmittel mit Aktivgehalten von 20, 25 bzw. 30 Gew.-% erhältlich sind. Es werden daher 9 Weichspülmittel folgender Zusammensetzung hergestellt:
◆ Mit 20 Gew.-% Aktivgehalt (Angaben in Gew.-%):

| | **Weichspülmittel** | | |
|---|---|---|---|
| | **1** | **2** | **3** |
| Mischung 1 | 22,2 | - | - |
| Mischung 2 | - | 22,2 | - |
| Mischung 6 | - | - | 22,2 |
| 25%ige wäßrige CaCl₂-Lösung | 1,2 | 1,2 | 1,2 |
| Parfüm | 0,4 | 0,4 | 0,4 |
| Rest zu 100 Gew.-% Wasser | | | |

◆ Mit 25 Gew.-% Aktivgehalt (Angaben in Gew.-%):

| | **Weichspülmittel** | | |
|---|---|---|---|
| | **4** | **5** | **6** |
| Mischung 1 | 27,8 | - | - |
| Mischung 2 | - | 27,8 | - |
| Mischung 6 | - | - | 27,8 |
| 25%ige wäßrige CaCl₂-Lösung | 3,2 | 3,2 | 3,2 |
| Parfüm | 0,4 | 0,4 | 0,4 |
| Rest zu 100 Gew.-% Wasser | | | |

◆ Mit 30 Gew.-% Aktivgehalt (Angaben in Gew.-%):

| | **Weichspülmittel** | | |
|---|---|---|---|
| | **7** | **8** | **9** |
| Mischung 1 | 33,3 | - | - |
| Mischung 2 | - | 33,3 | - |
| Mischung 6 | - | - | 33,3 |
| 25%ige wäßrige CaCl₂-Lösung | 4,0 | 4,0 | 4,0 |
| Parfüm | 0,5 | 0,5 | 0,5 |
| Rest zu 100 Gew.-% Wasser | | | |

Alle 9 Wäscheweichspülmittel werden einer Lagerstabilitätsprüfung im Klimaschrank unterzogen. Die Lagerstabilität wird im folgenden 24 Stunden Zyklus über einen Zeitraum von 14 Tagen im Klimaschrank "Prodicon" der Firma Weiss Umwelttechnik GmbH ermittelt: 11,5 Stunden bei 4 °C lagern, anschließend innerhalb von 30 Minuten auf 40 °C erwärmen; 11,5 Stunden bei 40 °C lagern und innerhalb von 30 Minuten auf 4 °C abkühlen.

Für die Beurteilung der Lagerstabilität wird die Viskosität bei 25 °C unmittelbar nach der Herstellung der Wäscheweichspülmittel sowie nach 1 und 2 Wochen Lagerung nach Brookfield (bei allen Messungen konstant 6 rpm, so daß bei niedrigen Viskositäten Spindel 1 und bei hohen Viskositäten Spindel 2 benutzt wird) bestimmt. Zur besseren Vergleichbarkeit wird über den gleichen Zeitraum eine Lagerung bei 20 °C durchgeführt. Im folgenden sind die gefundenen Viskositätswerte tabellarisch dargestellt (Angaben in mPa·s).

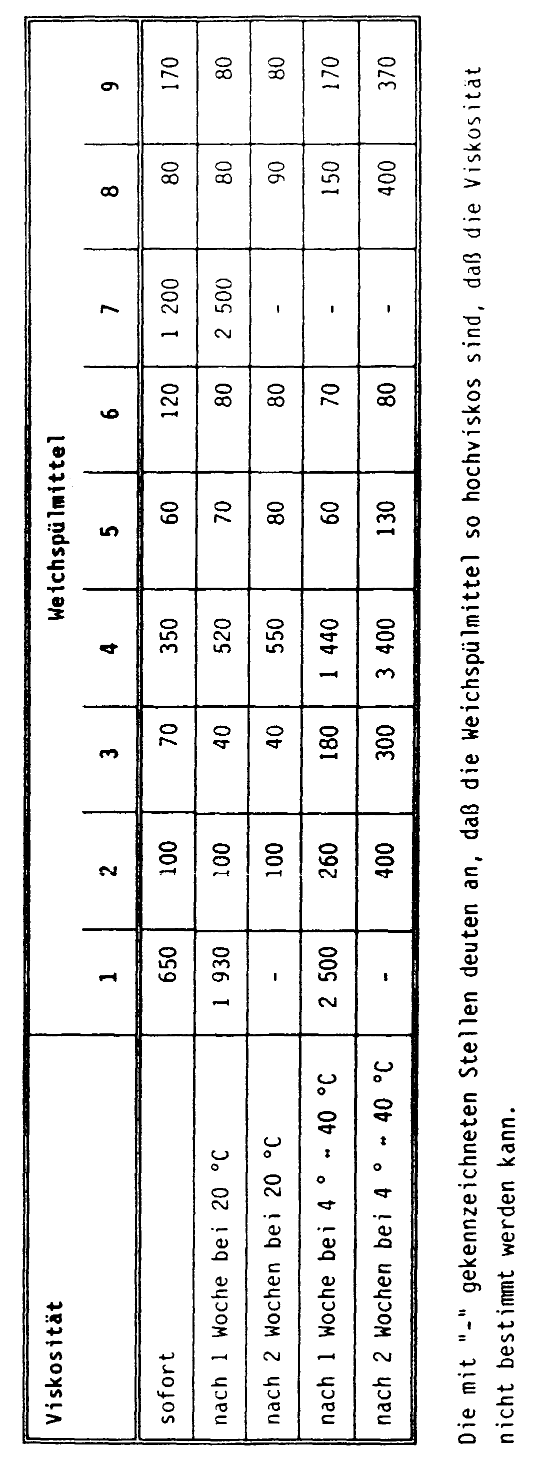

Unter den gewählten Versuchsbedingungen gilt ein Weichspülmittel als marktfähig, wenn die Viskosität nach 2 Wochen Lagerung im Klimaschrank höchstens 600 mPa·s beträgt. Die Weichspülmittel 1, 4 und 7, die alle auf Basis der Mischung 1 (= Verbindung 1) hergestellt worden sind, erfüllen diese Bedingung nicht. Sie sind extrem lagerinstabil. Anders verhält es sich mit den Weichspülmitteln 2, 5 und 8, die aus Mischung 2 hergestellt worden sind. Diese sind extrem lagerstabil. Das dafür nicht - wie vermutet - der höhere Anteil ungesättigter Acylreste in der Mischung 2 verantwortlich ist, belegen die ebenfalls extrem guten Lagereigenschaften der Weichspülmittel 3, 6 und 9. Diese basieren auf Mischung 6, die einen vergleichbaren Anteil ungesättigter Acylreste enthält, wie Mischung 1. Somit ist die hohe Lagerstabilität der Mischung 2 bzw. 6 enthaltenden Weichspülmittel auf die Mischungen aus 75 Gew.-% TEA-Quat auf Basis Talgfettsäure und 25 Gew.-% TEA-Esterquat auf Basis Talgfettsäuremethylester zurückzuführen.

Die Farbstabilität und textilweichmachende Wirkung der Mischungen 2 und 6, als auch die der daraus hergestellten Weichspülmittel 2, 3, 5, 6, 8 und 9 unterscheiden sich nicht von denen marktgängiger TEA-Quats bzw. daraus hergestellter Weichspülmittel.

## Patentansprüche

1. Quaternierte Triethanolamindifettsäureester erhalten durch Umsetzung von Triethanolamin mit 1,5 bis 2,5 Äquivalenten einer Mischung aus 70 bis 90 Gew.-% Fettsäure und 10 bis 30 Gew.-% Fettsäureester, jeweils bezogen auf die Gesamtmenge Fettsäure und Fettsäureester, und anschließender Quaternierung des Reaktionsproduktes mit einem Quaternierungsmittel, oder
durch getrennte Umsetzung von Triethanolamin mit 1,5 bis 2,5 Äquivalenten Fettsäure und Fettsäureester und Mischung der jeweils erhaltenen Triethanolamindifettsäureester vor der Quaternierung im Gewichtsverhältnis 70 bis 90 % zu 10 bis 30 % und anschließender Quaternierung, oder
durch getrennte Umsetzung von Triethanolamin mit 1,5 bis 2,5 Äquivalenten Fettsäure und Fettsäureester und Quaternierung mit einem Quaternierungsmittel und Mischung im Gewichtsverhältnis 70 bis 90 % zu 10 bis 30 %.

2. Quaternierte Triethanolamindifettsäureester nach Anspruch 1,
dadurch gekennzeichnet,
daß die Fettsäuren und die Fettsäurereste der Fettsäureester 6 bis 24 Kohlenstoffatome enthalten.

3. Quaternierte Triethanolamindifettsäureester nach mindestens einem der Ansprüche 1 und 2,
dadurch gekennzeichnet,
daß die Alkohole der Fettsäureester 1-bis 6-wertige Alkohole mit 1 bis 8 Kohlenstoffatomen sind.

4. Verfahren zur Herstellung von quaternierten Triethanolamidifettsäureestern aus Triethanolamin, Fettsäure, Fettsäureester und einem nierungsmittel,
dadurch gekennzeichnet,
daß entweder Triethanolamin mit 1,5 bis 2,5 Äquivalenten einer Mischung aus 70 bis 90 Gew.-% Fettsäure und 10 bis 30 Gew.-% Fettsäureester, jeweils bezogen auf die Gesamtmenge Fettsäure und Fettsäureester, umgesetzt und das Reaktionsprodukt anschließend quaterniert wird, oder
daß Triethanolamin mit 1,5 bis 2,5 Äquivalenten Fettsäure und Fettsäureester getrennt umgesetzt wird und dann die jeweils erhaltenen ethanolamindifettsäureester vor der Quaternierung im Gewichtsverhältnis 70 bis 90 % zu 10 bis 30 % gemischt und anschließend quaterniert werden, oder
daß Triethanolamin mit 1,5 bis 2,5 Äquivalenten Fettsäure und Fettsäureester getrennt umgesetzt wird und nach der jeweiligen Quaternierung im Gewichtsverhältnis 70 bis 90 % zu 10 bis 30 % gemischt wird.

5. Verwendung der nach Anspruch 4 hergestellten quaternierten Triethanolamindifettsäureester als Komponente in Wäscheweichspülmitteln.

## Claims

1. A quaternized triethanolamine difatty acid ester obtained by reacting triethanolamine with 1.5 to 2.5 equivalents of a mixture of 70 to 90% by weight of fatty acid and 10 to 30% by weight of fatty acid ester, each based on the total amount of fatty acid and fatty acid ester, and subsequent quaternization of the reaction product with a quaternizing agent, or by separate reaction of triethanolamine with 1.5 to 2.5 equivalents of fatty acid and fatty acid ester and mixing of the respectively obtained triethanolamine difatty acid esters before quaternization in a weight ratio of 70 to 90%:10 to 30% and subsequent quaternization, or by separate reaction of triethanolamine with 1.5 to 2.5 equivalents of fatty acid and fatty acid ester and quaternization with a quaternizing agent and mixing in a weight ratio of 70 to 90%:10 to 30%.

2. A quaternized triethanolamine difatty acid ester according to claim 1,
characterized in that
the fatty acids and the fatty acid radicals of the fatty acid esters contain 6 to 24 carbon atoms.

3. A quaternized triethanolamine difatty acid ester according to at least one of claims 1 and 2,
characterized in that
the alcohols of the fatty acid esters are 1- to 6-hydric alcohols having 1 to 8 carbon atoms.

4. A process for preparing quaternized triethanolamine difatty acid esters from triethanolamine, fatty acid, fatty acid esters and a quaternizing agent,
characterized in that
either triethanolamine is reacted with 1.5 to 2.5 equivalents of a mixture of 70 to 90% by weight of fatty acid and 10 to 30% by weight of fatty acid ester, each based on the total amount of fatty acid and fatty acid ester, and the reaction product is subsequently quaternized, or in that triethanolamine is separately reacted with 1.5 to 2.5 equivalents of fatty acid and fatty acid ester and the respectively obtained triethanolamine difatty acid esters are then mixed before quaternization in a weight ratio of 70 to 90%:10 to 30% and subsequently quaternized, or in that triethanolamine is separately reacted with 1.5 to 2.5 equivalents of fatty acid and fatty acid ester and, after the respective quaternization, mixed in a weight ratio of 70 to 90%:10 to 30%.

5. The use of the quaternized triethanolamine difatty acid esters prepared according to claim 4 in rinse cycle laundry softeners.

## Revendications

1. Esters de diacide gras de triéthanolamine quaternisés obtenus par réaction de triéthanolamine avec 1,5 à 2,5 équivalents d'un mélange à base de 70 à 90 % en poids d'acide gras et de 10 à 30 % en poids d'ester d'acide gras, respectivement rapporté à la quantité totale d'acide gras et d'ester d'acide gras, et quaternisation consécutive du produit de réaction avec un agent de quaternisation ou,
par réaction séparée de triéthanolamine avec 1,5 à 2,5 équivalents d'acide gras et d'ester d'acide gras et mélange de l'ester de diacide gras de triéthanolamine avant la quaternisation dans un rapport pondéral de 70 à 90 % pour 10 à 30 % et mélange dans un rapport pondéral de 70 à 90 % pour 10 à 30 %.

2. Esters de diacides gras de triéthanolamine quaternisés selon la revendication 1,
caractérisés en ce que
les acides gras et les radicaux d'acide gras des esters d'acide gras renferment de 6 à 24 atomes de carbone.

3. Esters de diacides gras de triéthanolamine quaternisés selon une des revendications 1 et 2,
caractérisés en ce que
les alcools des esters d'acide gras sont des alcools uni- à hexavalents ayant de 1 à 8 atomes de carbone.

4. Procédé de fabrication d'esters de diacides gras de triéthanolamine quaternisés, à partir de triéthanolamine, d'acides gras, d'ester d'acide gras et d'un agent de quaternisation,
caractérisé en ce qu'
on fait réagir soit la triéthanolamine avec de 1,5 à 2,5 équivalents d'un mélange à base de 70 à 90 % en poids d'acide gras et de 10 à 30 % en poids d'ester d'acide gras, respectivement rapporté à la quantité totale d'acide gras et d'ester d'acide gras, et on quaternise ensuite le produit de réaction, soit on fait réagir la triéthanolamine séparément avec de 1,5 à 2,5 équivalents d'acide gras et d'ester d'acide gras et ensuite on mélange l'ester de diacide gras de triéthanolamine avant la quaternisation dans un rapport pondéral de 70 à 90 % pour 10 à 30 % et ensuite on les quaternise,
soit on fait réagir séparément la triéthanolamine avec 1,5 à 2,5 équivalents d'acide gras et d'ester d'acide gras et après la quaternisation respective, on les mélange dans un rapport pondéral de 70 à 90 % pour 10 à 30 %.

5. Utilisation des esters de diacide gras de triéthanolamine quaternisés fabriqués selon la revendication 4, comme composants dans les produits assouplissants pour le linge.
